# EUROPEAN PATENT SPECIFICATION

(11) **EP 1 313 853 B1**
(45) Date of publication and mention of the grant of the patent: **21.03.2012**
(21) Application number: 01947067.3
(22) Date of filing: 12.06.2001
(51) Int. Cl.: C12N 15/11, C12N 15/113, A61K 31/7088, A61K 31/7135, A61K 31/7048, A61K 39/395

(54) **MODULATION OF FAS AND FASL EXPRESSION BY A SYNTHETIC PHOSPHODIESTER OLIGONUCLEOTIDE AND AN ANTI-FAS ANTIBODY**
MODULIERUNG DER FAS UND FASL EXPRESSION DURCH EIN SYNTHETISCHES OLIGONUKLEOTID UND EINEN ANTI-FAS ANTIKÖRPER
MODULATION DE L'EXPRESSION DE FAS ET FASL PAR UN OLIGONUCLÉOTIDE PHOSPHODIESTER SYNTHÉTIQUE ET UN ANTICORPS ANTI-FAS

(30) Priority: 29.08.2000 US 228925 P; 12.12.2000 WO PCT/CA00/01467; 12.12.2000 US 735363; 02.02.2001 US 266229 P
(43) Date of publication of application: 28.05.2003
(73) Proprietor: Bioniche Life Sciences Inc., Belleville, Ontario K8N 5J2 (CA)
(72) Inventor: PHILLIPS, Nigel, C., Pointe-Claire, Quebec H9R 1J6 (CA); FILION, Mario, C., Laval, Quebec H4P 2R2 (CA)
(74) Representative: Dey, Michael
(86) International application number: PCT/CA2001/000845
(87) International publication number: WO 2002/018593

(56) References cited:
- WO-A-01/44465
- WO-A-94/08053
- WO-A-96/23508
- WO-A-97/20924
- WO-A-99/03998
- SCAGGIANTE BRUNA ET AL: "Human cancer cell lines growth inhibition by GTn oligodeoxyribonucleotides recognizing single-stranded DNA-binding proteins" EUROPEAN JOURNAL OF BIOCHEMISTRY, vol. 252, no. 2, 1 March 1998 (1998-03-01), pages 207-215, XP002168739 ISSN: 0014-2956 cited in the application
- SOLARY ERIC ET AL: "Role of the Fas/Fas-L system in the immune response to tumors and the resistance to cytotoxic drugs." BULLETIN DU CANCER (PARIS), vol. 85, no. 8, August 1998 (1998-08), pages 685-694, XP008008873 ISSN: 0007-4551
- READER S ET AL: "IDENTIFICATION OF NON-ANTISENSE PHOSPHODIESTER OLIGONUCLEOTIDES THAT INDUCE CELL CYCLE ARREST AND APOPTOSIS IN CANCER CELLS" CLINICAL CANCER RESEARCH, vol. 6, no. SUPPL, 2000, page 526 XP001002269 ISSN: 1078-0432
- MORASSUTTI C ET AL: "CORRELATION BETWEEN CYTOTOXIC EFFECT AND BINDING TO NUCLEAR PROTEINS OF OLIGOMERIC D(GT)N SEQUENCES IN HUMAN CANCER CCRF-CEM CELL LINE" MINERVA BIOTECNOLOGICA, vol. 7, no. 2, 1 June 1995 (1995-06-01), pages 176-181, XP000671788 ISSN: 1120-4826

## Description

### FIELD OF THE INVENTION

The present invention relates to compositions useful for modulating Fas and Fas ligand expression on cells and for modulating efficacy of therapeutic agents.

### BACKGROUND OF THE INVENTION

WO 01/44465 describes therapeutically useful synthetic oligonucleotides as well as the use thereof, inter alia, for the treatment of cancer.

The Fas (Apo-1, CD95) and Fas ligand (FasL, CD95L) system is one of the best-studied cell death systems. Fas is a type-I membrane protein abundantly expressed by cells in various tissues and particularly on activated T cells, heart cells, kidney cells and hepatocytes. FasL is a type-II transmembrane protein expressed particularly on activated T cells and natural killer cells (Nagata, S. Ann. Rev. Genet. 33:29, 1999), and is expressed constitutively in immune-privileged sites as, for example, the eye and testis (Griffith et al. Science 270:630, 1995).

Fas and FasL interactions (Fas/FasL) play an essential role in the regulation of immune cells and in the elimination of autoreactive cells (Sabelko-Downes et al. Curr. Opin. Immunol. 12:330, 2000). In addition, Fas/FasL mediates the killing of cancer cells and of virus-infected cells (Famularo et al. Med. Hypoth. 53:50, 1999; Owen-Schaub et al. Int. J. Oncol. 17:5, 2000). In contrast, FasL, expressed on cancer cells, may attack cells of the immune system (O'Connell, J. Exp. Med. 184:1075, 1996) or facilitate local tumor invasion by killing surrounding tissue (Yoong et al. Am. J. Pathol. 154:693, 1999). FasL, expressed on activated T cells, may also participate in tissue damage in fulminant hepatitis and in graft-versus-host disease (Kondo et al. Nature Med. 3:409,1997; Braun et al. J. Exp. Med. 183:657, 1996).

Binding of FasL to Fas, or cross-linking of Fas with agonistic antibodies, induces apoptosis (Nagata, S. Ann. Rev. Genet. 33:29, 1999) that results in cell death. Apoptosis is an active cellular death process characterized by distinctive morphological changes that include condensation of nuclear chromatin, cell shrinkage, nuclear disintegration, plasma membrane blebbing, and the formation of membrane-bound apoptotic bodies (Wyllie et al. Int. Rev. Cytol. 68:251, 1980). A molecular hallmark of apoptosis is degradation of cellular nuclear DNA into oligonucleosomal-length fragments as the result of activation of endogenous endonucleases (Wyllie A. Nature 284:555, 1980). Caspases (cysteine-aspartyl-specific proteases) have been implicated as key enzymes in the execution of the late stage of apoptosis. The binding of FasL to Fas activates a cascade of caspases via a FADD adaptor (Fas-associated protein with death domain), which leads to the cleavage of various cellular substrates and to DNA fragmentation (Nagata, S. Ann. Rev. Genet. 33:29, 1999).

Synthetic oligonucleotides are polyanionic sequences that can be internalized by cells (Vlassov et al. Biochim. Biophys. Acta 1197:95, 1994) and bind selectively to nucleic acids (Wagner, R. Nature: 372:333, 1994), to specific cellular proteins (Bates et al. J. Biol. Chem. 274:26369, 1999) and to specific nuclear proteins (Scaggiante et al. Eur. J. Biochem. 252:207, 1998), and inhibit cell proliferation. Proliferation is the culmination of the progression of a cell through the cell cycle, resulting in the division of one cell into two cells. Alterations in cell cycle progression occur in all cancers and may result from over-expression of genes, mutation of regulatory genes, or abrogation of DNA damage checkpoints (Hochhauser D. Anti-Cancer Chemotherapeutic Agents 8:903, 1997).

Synthetic phosphorothioate oligonucleotides containing unmethylated CpG dinucleotides flanked by two 5' purine and two 3' pyrimidine (CpG motifs) are reported to induce the synthesis of cytokines by macrophages and B cells, to increase the activity of NK cells and cytotoxic T lymphocytes, and to enhance T-Natl. Acad. Sci. USA93:2879, 1996; Lipford et al. Eur. J. Immunol. 27:2340, 1997). A 20 base synthetic CpG motif is reported to block Fas expression on activated B cells and to block apoptosis induced by anti-Fas monoclonal antibodies (Wang et al. Cell. Immunol. 180:162, 1997). Irradiation is reported to upregulate the expression of Fas on cancer cells (Sheard et al. Int J. Cancer 73:757,1997; Nishioka et al. Int. J. Mol. Med. 3:275, 1999).

The ability to modulate the Fas/FasL system has many clinical applications for use in diseases including, but not limited to, neoplastic autoimmune, degenerative and cardiovascular diseases. However, most prior Fas/FasL modulating agents have proven to be less than adequate in clinical applications. Moreover, many of these agents are inefficient, toxic or have significant adverse effects.

Therefore, there is a continuing need for novel compositions and methods that modulate the expression of Fas and FasL on cells. There is also a need for novel compositions and methods that modulate the efficacy of Fas and FasL modulatory agents on disease. There is also a need for novel compositions and methods that modulate the expression of Fas and FasL on cells in order to treat diseases in animals or humans associated with altered expression of Fas or FasL on cells.

### SUMMARY OF THE INVENTION

The present invention therefore relates to the use of a synthetic phosphodiester oligonucleotide sequence of 6 bases selected from the group consisting of SEQ ID NO: 1 to SEQ ID NO: 18 and an anti-Fas antibody for the manufacture of a medicament for the treatment of a disease selected from a neoplastic disease, an autoimmune disease, an inflammatory disease, a proliferative disease, a lymphoproliferative disease, a degenerative disease, a neurodegenerative disease, a cardiovascular disease, a graft rejection or an infection, whereby the anti-Fas antibody is for administration in a dose of from 0.003 to 3 mg/kg, and wherein the oligonucleotides sequence modulates the efficacy of the anti-Fas antibody.

The present invention further relates to a synthetic phosphodiester oligonucleotide sequence of 6 bases selected from the group consisting of SEQ ID NO: 1 to SEQ ID NO: 18 and an anti-Fas antibody for use for the treatment of a disease selected from a neoplastic disease, an autoimmune disease, an inflammatory disease, a proliferative disease, a lymphoproliferative disease, a degenerative disease, a neurodegenerative disease, a cardiovascular disease, a graft rejection or an infection, whereby the anti-Fas antibody is in a dose of from 0.003 to 3 mg/kg, and wherein the oligonucleotide sequence modulate the efficacy of the anti-Fas antibody.

The present invention fulfills these needs by providing a novel use employing compositions comprising a synthetic phosphodiester oligodeoxynucleotide (hereinafter, "sequence") selected from the group consisting of (GG)ₙ, (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof, wherein the total number of bases in the composition is between 2 and 10, preferably 4 to 8, more preferably 5 to 7, and most preferably 6, namely compositions comprising a synthetic phosphodiester oligonucleotide sequence of 6 bases selected from the group consisting of SEQ ID NO:1 to SEQ ID NO:18.

The present invention provides new uses for these compositions.

This composition is also combined with a pharmaceutically acceptable carrier, and is administered to an animal or a human having a disease in an amount effective to modulate Fas and FasL expression in order to treat the disease in the animal or the human. This composition is also administered to an animal or human to modulate the efficacy of Fas and FasL modulatory agents, comprising administration of the composition in an amount effective to modulate efficacy of Fas and FasL modulatory agents to modulate Fas and FasL, or to treat an animal or a human having a disease. A new use for this composition of the present invention is to modulate, and preferentially potentiate, the efficacy of a therapeutic agent to treat a disease, comprising administration of the composition in a pharmaceutically acceptable carrier in combination with a therapeutic agent, in an amount effective to modulate efficacy of the therapeutic agent administered to an animal or a human. The compositions of the present invention may be administered at any time before, during, or after administration of the therapeutic agent, in order to modulate the efficacy of the therapeutic agent. The compositions of the present invention may also be administered *in vitro,* for example to animal or human cells or tissues.

The composition used in the present invention comprises a sequence selected from the group consisting of (GG)ₙ, (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof provided the total number of bases in the composition is 6, namely a synthetic phosphodiester oligonucleotide sequence of 6 bases selected from the group consisting of SEQ ID NO:1 to SEQ ID NO:18. In another preferred embodiment, the composition of the present invention comprises this sequence of 6 bases in combination with a pharmaceutically acceptable carrier. The composition of the present invention comprises this sequence of 6 bases in combination with a therapeutic agent, namely an anti-Fas antibody and optionally with a pharmaceutically acceptable carrier. These compositions may be used in any of the methods described in the preceding paragraphs and throughout the specification.

The unexpected and surprising ability of the sequences of the present invention addresses an unfulfilled need in the medical arts and provides an important benefit for animals, including humans.

Herein described is a composition comprising a synthetic phosphodiester oligodeoxynucleotide (hereinafter, "sequence") selected from the group consisting of (GG)ₙ, (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof, wherein the total number of bases in the composition is 2 to 10.

Further described is a composition comprising a synthetic phosphodiester oligodeoxynucleotide (hereinafter, "sequence") selected from the group consisting of (GG)ₙ, (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof, wherein the total number of bases in the composition is 4 to 8.

Further described is a composition comprising a synthetic phosphodiester oligodeoxynucleotide (hereinafter, "sequence") selected from the group consisting of (GG)ₙ, (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof, wherein the total number of bases in the composition is 5 to 7.

Further described is a composition comprising a synthetic phosphodiester oligodeoxynucleotide (hereinafter, "sequence") selected from the group consisting of (GG)ₙ, (GT)ₙ, a(GT)ₙb, a(GA)ₙb, and a(OC)ₙb, wherein n is an integer between 1 and 3, and a and b are independently either none or one or more As, Cs, Gs, or Ts, or combinations thereof, wherein the total number of bases in the composition is 6.

Further described are compositions comprising any of the sequences described herein, in combination with a pharmaceutically acceptable carrier.

Herein provided are compositions comprising any of the sequences SEQ ID NO:1 to SEQ ID NO:18 in combination with a therapeutic agent, namely an anti-Fas antibody and optionally a pharmaceutically acceptable carrier.

Herein provided is the use of any of these compositions for the manufacture of a medicament.

An object of the present invention is to provide new uses for these compositions.

Herein described is a method to modulate the Fas expression on cells.

Further described is a method to modulate FasL expression on cells.

Further described is a method to modulate the Fas expression on immune cells.

Further described is a method to modulate FasL expression on immune cells.

Further described is a method to modulate Fas expression on cancer cells.

Further described is a method to modulate FasL expression on cancer cells.

Another object of the present invention is to provide a method to treat a disease in an animal, including a human.

Yet another object of the present invention is to provide a method to treat a neoplastic disease.

Still another object of the present invention is to provide a method to treat an autoimmune disease.

Another object of the present invention is to provide a method to treat an inflammatory disease.

Yet another object of the present invention is to provide a method to treat a proliferative disease

Another object of the present invention is to provide a method to treat a lymphoproliferative disease.

Yet another object of the present invention is to provide a method to treat a degenerative disease.

Still another object of the present invention is to provide a method to treat a neurodegenerative disease.

Another object of the present invention is to provide a method to treat a cardiovascular disease

Yet another object of the present invention is to provide a method to treat a graft rejection.

Another object of the present invention is to provide a method effective to treat an infection.

Yet another object of the present invention is to provide a method that modulates the effect of a therapeutic agent to treat disease.

Yet another object of the present invention is to provide a method that potentiates the effect of a therapeutic agent to treat disease.

Still another object of the present invention is to provide a method that potentiates the effect of a Fas modulating agent.

Another object of the present invention is to provide a method that potentiates the effect of a FasL modulating agent.

Yet another object of the present invention is to provide a method that potentiates the effect of an anti-neoplastic agent.

Another object of the present invention is to provide a method that potentiates the effect of an immunostimulatory agent

Still another object of the present invention is to provide a method that potentiates the effect of an immunosuppressive agent.

Another object of the present invention is to provide a method that potentiates the effect of an anti-inflammatory agent.

Further described is a composition that is simple to prepare.

Further described is a composition that is minimally toxic to the recipient

These and other objects, features and advantages of the present invention will become apparent after a review of the following detailed description of the disclosed embodiment and the appended claims.

### DETAILED DESCRIPTION

The present invention fulfills these needs by providing compositions and new methods of using these compositions. The compositions comprise a synthetic phosphodiester oligodeoxynucleotide (hereinafter, "sequence") selected from the group consisting of (GG)ₙ. (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof, wherein the total number of bases in the composition is between 2 and 10, preferably 4 to 8, more preferably 5 to 7, and most preferably 6, namely a synthetic phosphodiester oligonucleotide sequence of 6 bases selected from the group consisting of SEQ ID NO:1 to SEQ ID NO:18. The compositions of the present invention further comprise any of these sequences in combination with a pharmaceutically acceptable carrier. The compositions used in the present invention comprise any of these sequences in combination with a therapeutic agent, namely an anti-Fas antibody and optionally with a pharmaceutically acceptable carrier.

The present invention provides new uses for these compositions. This composition is combined with a pharmaceutically acceptable carrier, and is administered to an animal or a human an amount effective to modulate Fas and FasL expression in the animal or the human. This composition is also combined with a pharmaceutically acceptable carrier, and is administered to an animal or a human having a disease in an amount effective to modulate Fas and FasL expression in order to treat the disease in the animal or the human. This composition is also administered to an animal or human to modulate the efficacy of Fas and FasL modulatory agents, comprising administration of the composition in an amount effective to modulate efficacy of Fas and FasL modulatory agents to modulate Fas and FasL, or to treat an animal or a human having a disease. Another new use for this composition of the present invention is to modulate, and preferentially potentiate, the efficacy of a therapeutic agent to treat a disease, The synthetic phosphodiester oligonucleotide sequence in combination with a therapeutic agent, in an amount effective to modulate efficacy of the therapeutic agent, namely an anti-Fas antibody, is administered to an animal or a human. The composition of the present invention may be administered at any time before, during, or after administration of the therapeutic agent, in order to modulate the efficacy of the therapeutic agent.

The composition used in the present invention comprises a sequence selected from the group consisting of (GG)ₙ, (GT)ₙ, a(GT)ₙb, a(GA)ₙb, and a(GC)ₙb, wherein n is an integer between 1 and 3, and a and b are independently either none or one or more As, Cs, Gs, or Ts, or combinations thereof provided the total number of bases in the composition is 6, namely a synthetic phosphodiester oligonucleotide sequence of 6 bases selected from the group consisting of SEQ ID NO:1 to SEQ ID NO:18. In another preferred embodiment, the composition of the present invention comprises this sequence of 6 bases in combination with a pharmaceutically acceptable carrier. The composition of the present invention comprises this sequence of 6 bases in combination with a therapeutic agent, namely an anti-Fas antibody and optionally with a pharmaceutically acceptable carrier. These compositions may be used in any of the methods described in this patent application.

As used herein the word "sequence" refers to a synthetic phosphodiester oligodeoxynucleotide comprised of adenine (A), cytosine (C), guanine (G) and thymine (T), with a total number of bases of 2 to 10, preferably 4 to 8, more preferably 5 to 7 and most preferably 6.

As used herein, the word "expression" refers to the cell surface concentration of Fas or of FasL.

As used herein, the words "response" refers to upregulation (increase) or downregulation (decrease) of Fas or of Fas L expression.

As used herein, the word "modulates" refers to changes in the expression of Fas or of FasL. Such changes include upregulation and downregulation of Fas or of FasL expression. The word modulate is also employed to describe the ability of the novel sequences of the present invention to modulate the efficacy of therapeutic agents, including Fas and FasL modulating agents, to treat disease or to modulate Fas or FasL expression.

As used herein, the phrases "therapeutically effective", "effective amount" and "amount effective to" refer to an amount of a sequence effective to modulate the expression of Fas or of FasL.

As used herein, the word "disease" refers to a condition wherein bodily health is impaired.

As used herein, the phrase "therapeutic agent" is any agent approved by a regulatory agency of a country or a state government or listed in the U.S. Pharmacopoeia or other generally recognized pharmacopoeia for use to treat a disease in an animal, including a human.

As used herein, the word "antineoplastic" refers to preventing the development, maturation, proliferation or spread of cancer cells

As used herein, the word "potentiates" refers to a degree of synergism that is greater than additive.

As used herein, the word "synergism" refers to the coordinated action of two or more agents.

Administration of an effective amount of a sequence of the present invention to an animal, including a human, is a therapeutic treatment that prevents, treats or eliminates a disease including, but not limited to, neoplastic, autoimmune, proliferative, lymphoproliferative, degenerative, and cardiovascular disease; infection; inflammation; and, graft, tissue and cell rejection.

Compositions comprising one or more sequences and a pharmaceutically acceptable carrier are prepared by uniformly and intimately bringing into association the sequence and the pharmaceutically acceptable carrier. Compositions comprising one or more sequences, a therapeutic agent and a pharmaceutically acceptable carrier are prepared by uniformly and intimately bringing into association the sequence, the therapeutic agent and the pharmaceutically acceptable carrier. Pharmaceutically acceptable carriers include liquid carriers, solid carriers or both. Liquid carriers are aqueous carriers, non-aqueous carriers or both and include, but are not limited to, solutions, suspensions and emulsions. Emulsions include, but are not limited to, oil emulsions, water in oil emulsions, water-in-oil-in-water emulsions, site-specific emulsions, long-carriers are biological carriers, chemical carriers or both and include, but are not limited to, viral vector systems, plasmids, particles, microparticles, nanoparticles, microspheres, nanospheres, bacterial cell walls, minipumps, and biodegradable or non-biodegradable natural or synthetic polymers that allow for sustained release of the sequences.

Preferred aqueous carriers include, but are not limited to, water, saline and pharmaceutically acceptable buffers. Preferred non-aqueous carriers include, but are not limited to, a mineral oil or a neutral oil including, but not limited to, a diglyceride, a triglyceride, a phospholipid, a lipid, an oil and mixtures thereof, wherein the oil contains an appropriate mix of polyunsaturated and saturated fatty acids. Optionally, stabilizing agents and excipients may be included regardless of the pharmaceutically acceptable carrier used to present the sequence to the cells.

The therapeutic effectiveness of a sequence may be increased by methods including, but not limited to, chemically modifying the base, sugar or phosphate backbone, chemically supplementing or biotechnologically amplifying the sequences using bacterial plasmids containing the appropriate sequences, complexing the sequences to biological or chemical carriers or coupling the sequences to tissue-type or cell-type directed ligands or antibodies. The composition of the present invention comprises a composition comprising a sequence and a therapeutic agent, namely an anti-Fas antibody, wherein, when the sequence and the therapeutic agent are combined with a pharmaceutically acceptable carrier and administered to an animal or human having a disease, the sequence modulates and preferentially potentiates the effect of the therapeutic agent on the disease. Therapeutic agents in general include, but not limited to, anti-neoplastic, anti-inflammatory, anti-autoimmune, anti-degenerative, Fas modulating and FasL modulating agents, or any combination thereof, and radiation therapy, or a combination of radiation therapy with therapeutic agents. These therapeutic agents include, but are not limited to, biologicals, drugs, chemotherapeutic drugs, immunostimulants, immunomodulators, immunotherapeutics, anti-virals, antiinfectives, antibiotics, cytokines, antigens, antibodies, nucleic acids, vaccines, aptabases, nucleic acids, antisense nucleic acids, telomerase inhibitors, caspase biologically engineered and chemically synthesized agents, and agents that target cell death molecules for activation or inactivation.

Chemotherapeutic drugs include, but are not limited to, DNA damaging, DNA-alkylating, DNA-cross-linking, anti-tumor antibiotic, topoisomerase inhibiting, purine inhibiting, pyrimidine inhibiting, microtubule stabilizing, microtubule destabilizing, anti-metabolic, hormone antagonist, protein kinase inhibiting, HMG-CoA inhibiting, metaloproteinase inhibiting, CDK inhibiting, cyclin inhibiting, angiogenesis inhibiting, differentiation enhancing and molecular biologically modified viral, bacterial and extotoxic agents.

Routes of administration include, but are not limited to, oral, topical, subcutaneous, transdermal, subdermal, intra-muscular, intra-peritoneal, intravesical, intra-articular, intra-prostatic, intra-arterial, intra-venous, intra-dermal, intra-cranial, intra-lesional, intra-tumoral, intra-ocular, intra-pulmonary, intraspinal, placement within cavities of the body, nasal inhalation and impression into skin. It is to be understood that administration of the compositions of the present invention may occur *in vivo, ex vivo* or *in vitro.* For example, the compositions of the present invention may be administered to animal or human cells or tissues *in vitro*. Appropriate doses for in vitro administration are about 1 nM to 1 mM, preferably about 10 nM to 100 µM, and more preferably about 100 nM to 10 µM.

Depending on the route of administration, the volume per dose is preferably about 0.001 to 100 ml per dose, more preferably about 0.01 to 50 ml per dose and most preferably about 0.1 to 30 ml per dose. A sequence in a pharmaceutically acceptable carrier, or sequence plus therapeutic agent in a pharmaceutically acceptable carrier, can be administered in a single dose treatment, in multiple dose treatments or continuously infused on a schedule and over a period of time appropriate to the disease being treated, the condition of the recipient and the route of administration. Moreover, the therapeutic agent can be administered before, concurrently with, or after administration of the sequence.

Preferably, the amount of sequence administered per dose is from about 0.001 to 100 mg/kg, more preferably from about 0.01 to 10 mg/ml and most preferably from about 0.1 to 5 mg/kg. The sequence modulates and preferentially potentiates, the effect of the therapeutic agent. Preferably, the amount of therapeutic agent administered per dose is from about 0.001 to 1000 mg/m² or from about 0.01 to 1000 mg/kg, more preferably from about 0.01 to 500 mg/m² or about 0.01 to 500 mg/kg and most preferably from about 0.1 to 100 mg/m² or about 0.1 to 100 mg/kg. According to the invention, in one embodiment of a therapeutic agent, anti-Fas antibodies are employed and are administered in a dose of from about 0.003 to about 0.3 mg/kg, preferably 0.01 to about 0.1 mg/kg.

The particular sequence and the particular therapeutic agent administered, the amount per dose, the dose schedule and the route of administration should be decided by the practitioner using methods known to those skilled in the art and will depend on the type of disease, the severity of the disease, the location of the disease and other clinical factors such as the size, weight and physical condition of the recipient. In addition, *in vitro* assays may optionally be employed to help identify optimal ranges for sequence and for sequence plus therapeutic agent administration.

The following examples will serve to further illustrate the present invention without, at the same time, however, constituting any limitation thereof. On the contrary, it is to be clearly understood that resort may be had to various other embodiments, modifications, and equivalents thereof which, after reading the description herein, may suggest themselves to those skilled in the art without departing from the spirit of the invention.

### Example 1

### Preparation of sequences

Phosphodiester sequences were prepared by Sigma-Genosys (Woodlands, TX) using Abacus Segmented Synthesis Technology. Unless stated otherwise, the sequences were dispersed in autoclaved deionized water or in a pharmaceutically acceptable buffer such as, but not limited to, saline immediately prior to use.

### Example 2

### Cells

All cell lines were obtained from the American Type Culture Collection (ATCC, Rockville, MD) and were cultured in the medium recommended by the ATCC. Table 1 shows the cell lines, their origins and their properties.

**Table 1**

| Cell lines | | |
|---|---|---|
| CELL LINE | ORIGIN | PROPERTIES |
| JURKAT | Human T cell leukemia | Suspension tumor model Atypical multi-drug resistance associated with p190-MRP protein |
| UMUC-3 | Human bladder cancer | P-glycoprotein overexpression |
| T-24 | Human bladder cancer | p53 mutated |
| LNCaP | Human prostate cancer | Solid tumor model; metastatic TGF-beta 1 receptor-negative; androgen-dependent |
| OVCAR-3 | Human ovarian cancer | Solid tumor model; metastatic p53 mutated; p21/waf-1/Cip-1 deleted |
| SK-OV-3 | Human ovarian cancer | Solid tumor model; metastatic TGF-beta 1 receptor-negative; androgen-dependent |
| MCF-7 | Human breast cancer | Solid tumor model; non-metastatic Caspase 3-negative; estrogen-depend |

Peripheral blood mononuclear cells (hereinafter, "PBMCs") were isolated from human blood by Ficoll-Hypaque (Amersham Pharmacia Biotech, Baie d'Urfée, Québec, Canada) density gradient centrifugation.

Cancer cells and PBMCs were seeded in 6 well flat-bottom microplates and were maintained at 37°C in a 5% CO₂ atmosphere. Unless stated otherwise, 2 X 10⁵ cells/ml were incubated for 48 h with 0 µg/ml (control) or 100 µg/ml (5.5 µM) (treated) of the sequences.

### Example 3

### Measurement of Fas and of FasL at the cell surface

Fas and FasL expression were measured by flow cytometry using anti-Fas FITC-conjugated monoclonal antibodies and anti-FasL PE-conjugated monoclonal antibodies in a FACScalibur Flow Cytometer (Becton Dickinson, San Jose, CA, USA) using the CELLQuest program (Becton Dickinson).

Results are expressed as percentage (%) increase in the expression of Fas

### Example 4 (comparative)

### Modulation of Fas and FasL on Jurkat leukemia T cells

Jurkat human leukemia T cells are an atypical multi-drug resistant human Suspension tumor cell model. Jurkat T cells were incubated with the 6 base sequences shown in Table 2.

**Table 2**

| Percentage increase of Fas and of FasL on Jurkat human leukemia T cells | | |
|---|---|---|
| SEQUENCE | % INCREASE | |
| | Fas | FasL |
| TGTGTG SEQ ID NO:1 - (6 bases) | 52 | 93 |
| GTGTGT SEQ ED NO:2 - (6 bases) | 52 | 43 |
| TTTGTT SEQ ID NO:3 - (6 bases) | 258 | 497 |
| GGTGGG SEQ ID NO:4 - (6 bases) | 61 | 22 |
| GGGTGG SEQ ID NO:5 - (6 bases) | 150 | 145 |
| TTGTTT SEQ ID NO:6 - (6 bases) | 126 | 140 |
| AAGTAA SEQ ID NO:7 - (6 bases) | -4 | -21 |
| CCGTCC SEQ ID NO:8 - (6 bases) | 107 | 151 |
| TGGTTG SEQ ID NO:9 - (6 bases) | 362 | 952 |
| ATGTAT SEQ ID NO:10 - (6 bases) | 246 | 393 |
| CTGTCT SEQ ID NO:11 - (6 bases) | 203 | 413 |
| TCGTTC SEQ ID NO:12 - (6 bases) | 121 | 75 |
| GGTTGG SEQ ID NO:13 - (6 bases) | 99 | 86 |
| GGAAGG SEQ ID NO:14 - (6 bases) | 67 | 88 |
| GGCCGG SEQ ID NO:15 - (6 bases) | 75 | 41 |
| GGGGGG SBQ ID NO:16 - (6 bases) | 118 | 49 |
| GGGAGG SEQ ID NO:17 - (6 bases) | 77 | 91 |
| GGGCGG SEQ ID NO:18 - (6 bases) | 208 | 356 |

### Example 5 (comparative)

### Modulation of Fas and of FasL on PBMCs

PBMCs were incubated with the 6 base sequences shown in Table 3.

**Table 3**

| Percentage increase of Fas and of FasL on PBMCs | | |
|---|---|---|
| SEQUENCE | % INCREASE | |
| | Fas | FasL |
| TGTGTG SEQ ID NO:1 - (6 bases) | 126 | 260 |
| GTGTGT SEQ ID NO:2 - (6 bases) | 94 | 99 |
| TTTGTT SEQ ID NO:3 - (6 bases) | 1165 | 1528 |
| GGTGGG SEQ ID NO:4 - (6 bases) | 57 | 59 |
| GGGTGG SEQ ID NO:5 - (6 bases) | 3 | -20 |
| TTGTTT SEQ ID NO:6 - (6 bases) | 338 | 441 |
| AAGTAA SEQ ID NO:7 - (6 bases) | 13 | -26 |
| CCGTCC SEQ ID NO:28 - (6 bases) | 1046 | 1147 |
| TGGTTG SEQ ID NO:9 - (6 bases) | 1043 | 1322 |
| ATGTAT SEQ ID NO:10 - (6 bases) | 377 | 457 |
| CTGTCT SEQ ID NO:11 - (6 bases) | 310 | 476 |
| TCGTTC SEQ ID NO:12 - (6 bases) | 597 | 847 |
| GGTTGG SEQ ID NO:13 - (6 bases) | -3 | -32 |
| GGAAGG SEQ ID NO:14 - (6 bases) | 112 | 162 |
| GGCCGG SEQ ID NO: 15 - (6 bases) | 6 | -29 |
| GGGGGG SBQ ID NO:16 - (6 bases) | 38 | 42 |
| GGGAGG SEQ ID NO:17 - (6 bases) | 266 | 356 |
| GGGCGG SEQ ID NO:18 - (6 bases) | 523 | 850 |

### Example 6 (comparative)

### Modulation of Fas and of FasL on T-24 bladder cancer cells

T-24 bladder cancer cells are a p53 mutated human cell line T-24 cells (1.0 X 10⁵ cells/ml) were incubated with the 6 base sequences shown in Table 4.

**Table 4**

| Percentage increase of Fas and of FasL on T-24 bladder cancer cells | | |
|---|---|---|
| SEQUENCE | % INCREASE | |
| | Fas | FasL |
| TGTGTG SBQ ID NO:1 - (6 bases) | 84 | 213 |
| GTGTGT SEQ ID NO:2 - (6 bases) | 93 | 77 |
| TTTGTT SEQ ID NO:3 - (6 bases) | 279 | 549 |
| GGTGGG SEQ ID NO:4 - (6 bases) | 106 | 29 |
| GGGTGG SEQ ID NO:5 - (6 bases) | 80 | 13 |
| TTGTIT SEQ ID NO:6 - (6 bases) | 116 | 152 |
| AAGTAA SEQ ID NO:7 - (6 bases) | 16 | -10 |
| CCGTCC SEQ ID NO:8 - (6 bases) | 282 | 459 |
| TGGTTG SEQ ID NO:9 - (6 bases) | 180 | 397 |
| ATGTAT SEQ ID NO:10 - (6 bases) | 90 | 150 |
| CTGTCT SEQ ID NO:11 - (6 bases) | 107 | 165 |
| TCGTTC SEQ ID NO:12 - (6 bases) | 105 | 74 |
| GGTTGG SEQ ID NO:13 - (6 bases) | 75 | 25 |
| GGAAGG SEQ ID NO:14 - (6 bases) | 46 | 14 |
| GGCCGG SEQ ID NO:15 - (6 bases) | 56 | 18 |
| GGGGGG SEQ ID NO:16 - (6 bases) | 35 | -14 |
| GGGAGG SEQ ID NO:17 - (6 bases) | 124 | 75 |
| GGGCGG SEQ ID NO:18 - (6 bases) | 139 | 205 |

### Example 7 (comparative)

### Modulation of Fas and of FasL on UMUC-3 bladder cancer cells

UMUC-3 bladder cancer cells are a P-glycoprotein overexpressing human cell line. UMUC-3 cells (1.0 X 10⁵ cells/ml) were incubated with the 6 base sequences shown in Table 5.

**Table 5**

| Percentage increase of Fas and of FasL on UMUC-3 bladder cancer cells | | |
|---|---|---|
| SEQUENCE | % INCREASE | |
| | Fas | FasL |
| TGTGTG SEQ ID NO:1 - (6 bases) | 72 | 112 |
| GTGTGT SEQ ID NO:2 - (6 bases) | 72 | 154 |
| TTTGTT SEQ ID NO:3 - (6 bases) | 108 | 231 |
| GGTGGG SEQ ID NO:4 - (6 bases) | 62 | 113 |
| GGGTGG SEQ ID NO:5 - (6 bases) | 67 | 143 |
| TTGTTT SEQ ID NO:6 - (6 bases). | 57 | 132 |
| AAGTAA SEQ ID NO:7 - (6 bases) | 6 | -1 |
| CCGTCC SEQ ID NO:8 - (6 bases) | 55 | 149 |
| TGGTTG SEQ ID NO:9 - (6 bases) | 143 | 300 |
| ATGTAT SEQ ID NO:10 - (6 bases) | 42 | 95 |
| CTGTCT SEQ ID NO:11 - (6 bases) | 45 | 156 |
| TCGTTC SEQ ID NO:12 - (6 bases) | 47 | 112 |
| GGTTGG SEQ ID NO: 13-(6 bases) | 47 | 98 |
| GGAAGG SBQ ID NO: 14-(6 bases) | 1 | 8 |
| GGCCGG SEQ ID NO:15 - (6 bases) | 23 | 58 |
| GGGGGG SEQ ID NO: 16-(6 bases) | -5 | 10 |
| GGGAGG SEQ ID NO: 17-(6 bases) | 39 | 106 |
| GGGCGG SEQ ID NO:18-(6 bases) | 72 | 250 |

### Example 8 (comparative)

### Modulation of Fas and of FasL on OVCAR-3 ovarian cancer cells

OVCAR-3 ovarian cancer cells are a p53 mutated, p21/waf-1/Cip deleted, metastatic human solid tumor model. OVCAR-3 cells were incubated with the 6 base sequences shown in Table 6.

**Table 6**

| Percentage increase of Fas and of FasL on OVCAR-3 ovarian cancer cells | | |
|---|---|---|
| SEQUENCE | % INCREASE | |
| | Fas | FasL |
| TGTGTG SEQ ID NO:1 - (6 bases) | 64 | 70 |
| GTGTGT SEQ ID NO:2 - (6 bases) | 77 | 69 |
| TTTGTT SEQ ID NO:3 - (6 bases) | 193 | 341 |
| GGTGGG SEQ ID NO:4 - (6 bases) | 41 | 14 |
| GGGTGG SEQ ID NO:5 - (6 bases) | 43 | 9 |
| TTGTIT SEQ ID NO:6 - (6 bases) | 65 | 98 |
| AAGTAA SEQ ID NO:7 - (6 bases) | 16 | -2 |
| CCGTCC SEQ ID NO: 8-(6 bases) | 83 | 95 |
| TGGTTG SEQ ID NO:9 - (6 bases) | 221 | 270 |
| ATGTAT SEQ ID NO:10 - (6 bases) | 93 | 114 |
| CTGTCT SEQ ID NO: 11 - (6 bases) | 52 | 93 |
| TCGTTC SEQ ID NO: 12 - (6 bases) | 126 | 224 |
| GGTTGG SEQ ID NO:13 - (6 bases) | 22 | 10 |
| GGAAGG SEQ ID NO:14 - (6 bases) | 15 | 1 |
| GGCCGG SEQ ID NO:15 - (6 bases) | 19 | -2 |
| GGGGGG SEQ ID NO:16 - (6 bases) | -1 | 7 |
| GGGAGG SEQ ID NO:17 - (6 bases) | 49 | 59 |
| GGGCGG SEQ ID NO: 18 - (6 bases) | 65 | 142 |

### Example 9 (comparative)

### Modulation of Fas and of FasL on SK-OV 3 ovarian cancer cells

SK-OV-3 ovarian cancer cells are a p53 mutated, p21/waf-1/Cip deleted, p15^{ink4B},p16^{ink4} deleted metastatic human solid tumor model. SK-OV 3 cells (1.0 X 10⁵ cells/ml) were incubated with the 6 base sequences shown in Table 7.

**Table 7**

| Percentage increase of Fas and FasL on SK-OV-3 ovarian cancer cells | | |
|---|---|---|
| SEQUENCE | % INCREASE | |
| | Fas | FasL |
| TGTGTG SEQ ID NO:1 - (6 bases) | 49 | 37 |
| GTGTGT SEQ ID NO:2 - (6 bases) | 60 | 24 |
| TTTGTT SEQ ID NO:3 - (6 bases) | 108 | 103 |
| GGTGGG SEQ ID NO:4 (6 bases) | -1 | -17 |
| GGGTGG SEQ ID NO:5 - (6 bases) | 3 | -31 |
| TTGTTT SEQ ID NO:6 - (6 bases) | 41 | 24 |
| AAGTAA SEQ ID NO:7 - (6 bases) | -12 | -22 |
| CCGTCC SEQ ID NO:8 - (6 bases) | 58 | 32 |
| TGGTTG SEQ ID NO:9 - (6 bases) | 63 | 53 |
| ATGTAT SEQ ID NO: 10 - (6 bases) | 34 | 33 |
| CTGTCT SEQ ID NO:11 - (6 bases) | 15 | 26 |
| TCGTTC SEQ ID NO:12 - (6 bases) | 9 | -3 |
| GGTTGG SEQ ID NO: 13 - (6 bases) | 16 | -3 |
| GGAAGG SEQ ID NO:14 - (6 bases) | -15 | -31 |
| GGCCGG SEQ ID NO:15 - (6 bases) | 0 | -27 |
| GGGGGG SEQ ID NO:16 - (6 bases) | -14 | -28 |
| GGGAGG SEQ ID NO:17- (6 bases) | -6 | -16 |
| GGGCGG SEQ ID NO:18 - (6 bases) | 29 | 42 |

### Example 10 (comparative)

### Modulation of Fas and of FasL on LNCaP prostate cancer cells

LNCaP prostate cancer cells are a TGF-beta 1 receptor negative, androgen-independent, metastatic human solid tumor model. LNCaP cells were incubated with the 6 base sequences shown in Table 8.

**Table 8**

| Percentage increase of Fas and of FasL on LNCaP prostate cancer cells | | |
|---|---|---|
| SEQUENCE | % INCREASE | |
| | Fas | FasL |
| TGTGTG SEQ ID NO:1 - (6 bases) | 29. | 40 |
| GTGTGT SEQ ID NO:2 - (6 bases) | 34 | 5 |
| TTTGTT SEQ ID NO:3 - (6 bases) | 199 | 344 |
| GGTGGG SEQ ID NO:4 - (6 bases) | 21 | 8 |
| GGGTGG SEQ ID NO:5 - (6 bases) | 15 | -1 |
| TTGTTT SEQ ID NO:6 - (6 bases) | 40 | 73 |
| AAGTAA SEQ ID NO:7 - (6 bases) | 0 | -16 |
| CCGTCC SEQ ID NO:8 - (6 bases) | 0 | 311 |
| TGGTTG SEQ ID NO:9 - (6 bases) | 184 | 241 |
| ATGTAT SEQ ID NO:10 - (6 bases) | 44 | 68 |
| CTGTCT SEQ ID NO:11 - (6 bases) | 17 | 55 |
| TCGTTC SEQ ID NO: 12 - (6 bases) | 111 | 171 |
| GGTTGG SEQ ID NO:13 - (6 bases) | 23 | -3 |
| GGAAGG SEQ ID NO:14 - (6 bases) | 26 | -4 |
| GGCCGG SEQ ID NO: 15 - (6 bases) | 12 | -4 |
| GGGGGG SEQ ID NO:16 - (6 bases) | 6 | 13 |
| GGGAGG SEQ ID NO:17 - (6bases) | 48 | 55 |
| GGGCGG SEQ ID NO: 18 - (6 bases) | 55 | 126 |

### Example 11 (comparative)

### Modulation of Fas and of FasL on MCF-7 breast cancer cells

MCF-7 human breast cancer cells are a caspase-3 negative, estrogen-dependent human solid tumor model. MCF-7 cells (1 x 10⁵ cells/ml) were incubated with the 6 base sequences shown in Table 9.

**Table 9**

| Percentage increase of Fas and of FasL on MCF-7 breast cancer cells | | |
|---|---|---|
| SEQUENCE | % INCREASE | |
| | Fas | FasL |
| TGTGTG SEQ ID NO:1 - (6 bases) | 122 | 121 |
| GTGTGT SEQ ID NO:2 - (6 bases) | 155 | 135 |
| TTTGTT SEQ ID NO:3 - (6 bases) | 361 | 528 |
| GGTGGG SEQ ID NO:4 - (6 bases) | 106 | 110 |
| GGGTGG SEQ ID NO:5 - (6 bases) | 93 | 81 |
| TTGTTT SEQ ID NO:6 - (6 bases) | 143 | 159 |
| AAGTAA SEQ ID NO:7 - (6 bases) | 12 | 21 |
| CCGTCC SEQ ID NO:8 - (6 bases) | 200 | 322 |
| TGGTTG SEQ ID NO:9 - (6 bases) | 255 | 481 |
| ATGTAT SEQ ID NO:10 - (6 bases) | 129 | 214 |
| CTGTCT SEQ ID NO:11 - (6 bases) | 73 | 128 |
| TCGTTC SEQ ID NO:12 - (6 bases) | 56 | 98 |
| GGTTGG SEQ ID NO:13 - (6 bases) | 99 | 83 |
| GGAAGG SEQ ID NO:14 - (6 bases) | 62 | 84 |
| GGCCGG SEQ ID NO:15 - (6 bases) | 63 | 78 |
| GGGGGG SEQ ID NO:16 - (6 bases) | -7 | 18 |
| GGGAGG SEQ ID NO: 17-(6 bases) | 127 | 194 |
| GGGCGG SEQ ID NO:18 - (6 bases) | 165 | 322 |

### Example 12 (comparative)

### Inhibition of Fas expression on Jurkat human leukemia T cells by cycloheximicle

Jurkat human leukemia T cells were pre-incubated with 0.0 µg/ml (- CHX) or with 0.1 µg/ml of cycloheximide (+ CHX) for 1 h. Ten µg/ml or 100 µg/ml of 6 base SEQ ID NO:5 (GGGTGG) was added to both the - CHX and + CHX cells and the incubation was continued for 24 hours and for 48 hours (Table 10).

**Table 10**

| Percentage increase of Fas on Jurkat human leukemia T cells | | | | |
|---|---|---|---|---|
| | % INCREASE | | | |
| SEQUENCE | 24 h | | 48 h | |
| | - CHX | + CHX | - CHX | + CHX |
| GGGTGG SEQ ID NO:5 - (6 bases) 10 µg/ml | 63 | 5 | 91 | 51 |
| GGGTGG SEQ ID NO:5 -(6 bases) 100 µg/ml | 62 | 22 | 150 | 115 |

As shown in Table 10, SEQ ID NO:5 upregulated Fas expression on Jurkat T cells. With 10 µg/ml of SEQ ID NO:5, cycloheximide decreased Fas expression 92% after 24 h and 44% after 48 h. With 100 µg/ml of SEQ ID NO:5 cycloheximide decreased Fas expression 65% after 24 h and 23% after 48 h. These data suggest that 6 base SEQ ID NO:5 stimulates de novo synthesis of Fas.

### Example 13

### Synergistic effect of SEQ ID No:5 (GGGTGG) and agonistic anti-Fas antibodies on inhibition of UMUC-3 bladder cancer cell proliferation

UMUC-3 bladder cancer cells were incubated for 48 hours with 0.00, 0.02 and 0.20 µg/ml of agonistic anti-Fas monoclonal antibodies (clone CH-11: Coulter-Immunotech, Marseille, France) + 0 or 10 µg/ml of SEQ ID NO:5. Cell proliferation was measured using dimethylthiazol-diphenyltetrazolium (MTT) reduction (Mosman et al. J. Immunol. Methods 65:55, 1983). MTI was measured at a wavelength of 570 nm using a multiplate spectrophotometer reader (ELX800, Bio-TEK Instruments Inc., Winooski, VT).

**Table 11**

| Inhibition of UMUC-3 bladder cancer cell proliferation | | | |
|---|---|---|---|
| SEQUENCE | % INHIBITION | | |
| | Anti-Fas antibodies | | |
| | 0.0 µg/ml | 0.02 µg/ml | 0.2 µg/ml |
| No sequence | 0 | 7 | 17 |
| GGGTGG SEQ ID NO:5 - (6 bases) 10 µg/ml | 23 | 38 | 44 |

As shown in Table 11, 6 base SEQ ID NO:5 potentiated the inhibitory activity of 0.02 and 0.2 µg/ml of agonistic anti-Fas antibodies on UMUC-3 cell proliferation.

### Example 14

### Additive effect of SEQ ID NO:5 (GGGTGG) and agonistic anti-Fas antibodies on inhibition of Jurkat leukemia T cell proliferation.

Jurkat T leukemia cells were incubated for 48 hours with 0.00, 0.02 and 0.20 µg/ml of agonistic auti-Fas monoclonal antibodies (clone CH-11) + 0 and 10 µg/ml of SEQ ID NO:5. Cell proliferation was measured as in Example 13.

**Table 12**

| Inhibition of Jurkat human leukemia T cell proliferation | | | |
|---|---|---|---|
| SEQUENCE | % INHIBITION | | |
| | Anti-Fas antibodies | | |
| | 0.0 µg/ml | 0.02 µg/ml | 0.2 µg/ml |
| No sequence | 0 | 16 | 33 |
| GGGTGG SEQ ID NO:5 - (6 bases) 10 µg/ml | 16 | 28 | 48 |

As shown in Table 12, the effects of 6 base SEQ ID NO:5 and of 0.02 and 0.2 µg/ml of agonistic anti-Fas antibodies on Jurkat T cell proliferation were additive.

### Example 15

### Effect of 6 base sequences and agonistic anti-Fas antibodies on EL-4 murine T

EL-4 murine T lymphoma cells are implanted into C57BL6 lpr/Ipr (Fas negative mice). The mice are divided into 30 groups of 10 mice. On day 0, groups 1 mice receive saline, group 2 mice receive 1,10 or 100 mg/kg of SEQ. ID NO:5, group 3 mice receive 1,10 or 100 mg/kg of SEQ. ID NO: 16, group 4 receive 1,10 or 100 mg/kg of SEQ. ID NO:17, group 5 mice receive 1, 10 or 100 mg/kg of SEQ. ID NO:18, group 6 mice receive anti-Fas antibodies, group 7 mice receive 1, 10 or 100 mg/kg of SEQ. ID NO: 5 + anti-Fas antibodies, group 8 mice receive 1, 10 or 100 mg/kg of SEQ. ID NO:16 + anti-Fas antibodies, group 9 receive 1, 10 or 100 mg/kg of SEQ. ID NO:17 + anti-Fas antibodies, group 10 mice receive 1_{;} 10 or 100 mg/kg of SEQ. ID NO:18 + anti-Fas antibodies. In these different groups, anti-Fas antibodies are administered at any dose in the range of about 0.003 to about 0.3 mg/kg. Group 1 mice have the most tumor mass, groups 2, 3, 4, 5 and 6 mice have less tumor mass than group 1 mice, and group 7, 8, 9 and 10 mice have the least tumor mass. The efficacy of the SEQ. ID NO:5, the SEQ. ID NO:16 and the SEQ. ID NO:17 is dose-dependent

### Example 16

Female (SJL/J x PL/J) F1 mice are injected subcutaneously in both femoral regions with an emulsion containing 0.5 mg of myelin basic protein (MBP) mixed with complete Freund's adjuvant. After 24 h, 400 ng of *Bordetella pertussis* toxin is administered intraperitoneally. On the day of onset (day 0), group 1 mice receive saline, group 2 mice receive 1, 10 or 100 mg/kg of SEQ. ID NO:5, group 3 mice receive 1, 10 or 100 mg/kg of SEQ. ID NO:16, group 4 mice receive 1, 10 or 100 mg/kg of SEQ. ID NO:17, group 5 mice receive 1, 10 or 100 mg/kg of SEQ. ID NO:18, group 6 mice receive anti-Fas antibodies, group 7 mice receive 1, 10 or 100 mg/kg of SEQ. ID NO:5 + anti-Fas antibodies, group 8 mice receive 1, 10 or 100 mg/kg of SEQ. ID NO:16 + anti-Fas antibodies, group 9 receive 1, 10 or 100 mg/kg of SEQ. ID NO:17 + anti-Fas antibodies, group 10 mice receive 1, 10 or 100 mg/kg of SEQ. ID NO:18 + anti-Fas antibodies for 3 days by intracisternal administration (20 µg/day). In these different groups, anti-Fas antibodies are administered at any dose in the range of about 0.003 to about group 1, 2, 3, 4 and 5 mice. Group 7, 8, 9 and 10 mice show the least progression of EAE. The efficacy of the SEQ. ID NO:5, the SEQ. ID NO:16 and the SEQ. ID NO:17 is dose-dependent

## Claims

1. Use of a synthetic phosphodiester oligonucleotide sequence of 6 bases selected from the group consisting of SEQ ID NO: 1 to SEQ ID NO: 18 and an anti-Fas antibody for the manufacture of a medicament for the treatment of a disease selected from a neoplastic disease, an autoimmune disease, an inflammatory disease, a proliferative disease, a lymphoproliferative disease, a degenerative disease, a neurodegenerative disease, a cardiovascular disease, a graft rejection or an infection, whereby the anti-Fas antibody is for administration in a dose of from 0.003 to 3 mg/kg, and wherein the oligonucleotide sequence modulates the efficacy of the anti-Fas antibody.

2. Use according to claim 1. whereby the anti-Fas antibody is for administration in a dose of from 0.01 to 0.1 mg/kg.

3. Use according to claim 1 or 2, for the manufacture of a medicament for the treatment of cancer.

4. Use according to claim 3, wherein the cancer is selected from the group consisting of ovarian, prostate, breast, bladder and leukemia.

5. Use according to any one of claims 1 to 4 of a synthetic phosphodiester oligonucleotide sequence of 6 bases selected from the group consisting of SEQ ID NO: 5, SEQ ID NO: 16, SEQ ID NO: 17 and SEQ ID NO: 18 and an anti-Fas antibody for the manufacture of a medicament for the treatment of cancer.

6. A synthetic phosphodiester oligonucleotide sequence of 6 bases selected from the group consisting of SEQ ID NO: 1 to SEQ ID NO: 18 and an anti-Fas antibody for use for the treatment of a disease selected from a neoplastic disease, an autoimmune disease, an inflammatory disease, a proliferative disease, a lymphoproliferative disease, a degenerative disease, a neurodegenerative disease, a cardiovascular disease, a graft rejection or an infection, whereby the anfi-Fas antibody is in a dose of from 0.003 to 3 mg/kg, and wherein the oligonucleotide sequence modulates the efficacy of the anti-Fas antibody.

## Patentansprüche

1. Verwendung einer synthetischen Phosphodiester-Oligonukleotidsequenz aus 6 Basen, ausgewählt aus der Gruppe bestehend aus SEQ ID NR: 1 bis SEQ ID NR: 18 und einem anti-Fas Antikörper für die Herstellung eines Medikamentes für die Behandlung einer Krankheit ausgewählt aus einer neoplastischen Erkrankung, einer Autoimmunerkrankung, einer Entzündungserkrankung, einer proliferativen Erkrankung, einer lymphoproliferativen Erkrankungen, einer degenerativen Erkrankung, einer neurodegenerativen Erkrankung, einer kardiovaskulären Erkrankung, Transplantatabstoßung oder einer Infektion, wobei der anti-Fas Antikörper für die Verabreichung in einer Dosis von 0,003 bis 3 mg/kg ist und wobei die Oligonukleotidsequenz die Effizienz des anti-Fas Antikörpers moduliert.

2. Verwendung nach Anspruch 1, wobei der anti-Fas Antikörper zur Verabreichung in einer Dosis von 0,01 bis 0,1 mg/kg ist.

3. Verwendung nach Anspruch 1 oder 2 für die Herstellung eines Medikamentes zur Behandlung von Krebs.

4. Verwendung nach Anspruch 3, wobei die Krebserkrankung ausgewählt ist aus der Gruppe bestehend aus Eierstock, Prostata, Brust, Blase und Leukämie.

5. Verwendung nach einem der Ansprüche 1 bis 4 einer synthetischen Phosphodiester-Oligonukleotidsequenz aus 6 Basen, ausgewählt aus der Gruppe bestehend aus SEQ ID NR: 5, SEQ ID NR: 16, SEQ ID NR: 17 und SEQ ID NR: 18 und einem anti-Fas Antikörper für die Herstellung eines Medikamentes für die Behandlung von Krebs.

6. Eine synthetischen Phosphodiester-Oligonukleotidsequenz aus 6 Basen, ausgewählt aus der Gruppe bestehend aus SEQ ID NR: 1 bis SEQ ID NR: 18 und einem anti-Fas Antikörper für die Verwendung zur Behandlung einer Krankheit ausgewählt aus einer neoplastischen Erkrankung, einer Autoimmunerkrankung, einer Entzündungserkrankung, einer proliferativen Erkrankung, einer lymphoproliferativen Erkrankungen, einer degenerativen Erkrankung, einer neurodegenerativen Erkrankung, einer kardiovaskulären Erkrankung, Transplantat Abstoßung oder einer Infektion, wobei der anti-Fas Antikörper in einer Dosis von 0,003 bis 3 mg/kg ist und wobei die Oligonukleotidsequenz die Effizienz des anti-Fas Antikörpers moduliert.

## Revendications

1. Utilisation d'une séquence oligonucléotidique à phosphodiesters synthétique de 6 bases choisie dans le groupe consistant en SEQ ID NO :1 à SEQ ID NO : 18 et d'un anticorps anti-Fas pour la fabrication d'un médicament pour le traitement d'une maladie choisie parmi une maladie néoplasique, une maladie auto-immune, une maladie inflammatoire, une maladie proliférative, une maladie lymphoproliférative, une maladie dégénérative, une maladie neurodégénérative, une maladie cardiovasculaire, un rejet de greffe ou une infection, où l'anticorps anti-Fas est destiné à l'administration en une dose de 0,003 à 3 mg/kg, et où la séquence oligonucléotidique module l'efficacité de l'anticorps anti-Fas.

2. Utilisation selon la revendication 1 où l'anticorps anti-Fas est destiné à l'administration en une dose de 0,01 à 0,1 mg/kg.

3. Utilisation selon la revendication 1 ou 2 pour la fabrication d'un médicament pour le traitement d'un cancer.

4. Utilisation selon la revendication 3 où le cancer est choisi dans le groupe consistant en le cancer ovarien, de la prostate, du sein, de la vessie et la leucémie.

5. Utilisation selon l'une quelconque des revendications 1 à 4 d'une séquence oligonucléotidique à phosphodiesters synthétique de 6 bases choisie dans le groupe consistant en SEQ ID NO : 5, SEQ ID NO : 16, SEQ ID NO : 17 et SEQ ID NO : 18 et d'un anticorps anti-Fas pour la fabrication d'un médicament pour le traitement d'un cancer.

6. Séquence oligonucléotidique à phosphodiesters synthétique de 6 bases choisie dans le groupe consistant en SEQ ID NO :1 à SEQ ID NO: 18 et anticorps anti-Fas destinés à être utilisés pour le traitement d'une maladie choisie parmi une maladie néoplasique, une maladie auto-immune, une maladie inflammatoire, une maladie proliférative, une maladie lymphoproliférative, une maladie dégénérative, une maladie neurodégénérative, une maladie cardiovasculaire, un rejet de greffe ou une infection, où l'anticorps anti-Fas est en une dose de 0,003 à 3 mg/kg, et où la séquence oligonucléotidique module l'efficacité de l'anticorps anti-Fas.
